# EUROPEAN PATENT SPECIFICATION

(11) **EP 3 870 274 B1**
(45) Date of publication and mention of the grant of the patent: **13.09.2023**
(21) Application number: 19876581.0
(22) Date of filing: 22.10.2019
(51) Int. Cl.: A61N 1/36, A61B 5/24, A61B 5/00, A61N 1/05

(54) **DEVICE FOR CONTROLLED NEURAL STIMULATION**
VORRICHTUNG ZUR GESTEUERTEN NEURONALEN STIMULATION
DISPOSITIF POUR STIMULATION NEURONALE CONTRÔLÉE

(30) Priority: 23.10.2018 AU 2018904011
(43) Date of publication of application: 01.09.2021
(73) Proprietor: Saluda Medical Pty Ltd, Artarmon, New South Wales 2064 (AU)
(72) Inventor: PARKER, John Louis, Artarmon, NSW 2064 (AU); GORMAN, Robert Bruce, Artarmon, NSW 2064 (AU)
(74) Representative: Abel & Imray LLP
(86) International application number: PCT/AU2019/051151
(87) International publication number: WO 2020/082118

(56) References cited:
- WO-A1-2016/048974
- WO-A1-2019/231796
- US-A1- 2006 129 205
- US-A1- 2007 225 765
- US-A1- 2007 265 489
- US-A1- 2014 277 267
- US-A1- 2017 173 341
- US-A1- 2018 104 493
- US-A1- 2019 030 339
- US-B2- 8 401 655

## Description

### Technical Field

The present invention relates to applying electrical neural stimuli in order to give rise to a compound action potential upon a nerve, and in particular the present invention relates to configuring a stimulus in order to controllably evoke a compound action potential having an expected morphology.

### Background of the Invention

There are a range of situations in which it is desirable to apply neural stimuli in order to give rise to a compound action potential (CAP). For example, neuromodulation is used to treat a variety of disorders including chronic pain, Parkinson's disease, and migraine. A neuromodulation system applies an electrical pulse to tissue in order to generate a therapeutic effect. When used to relieve chronic pain, the electrical pulse is applied to the dorsal column (DC) of the spinal cord, referred to as spinal cord stimulation (SCS). Neuromodulation systems typically comprise an implanted electrical pulse generator, and a power source such as a battery that may be rechargeable by transcutaneous inductive transfer. An electrode array is connected to the pulse generator, and is positioned in the dorsal epidural space above the dorsal column. An electrical pulse applied to the dorsal column by an electrode causes the depolarisation of neurons, and generation of propagating action potentials. The fibres being stimulated in this way inhibit the transmission of pain from that segment in the spinal cord to the brain. To sustain the pain relief effects, stimuli are applied substantially continuously, for example at a frequency in the range of 50-100 Hz.

Neuromodulation may also be used to stimulate efferent fibres, for example to induce motor functions. In general, the electrical stimulus generated in a neuromodulation system triggers a neural action potential which then has either an inhibitory or excitatory effect. Inhibitory effects can be used to modulate an undesired process such as the transmission of pain, or to cause a desired effect such as the contraction of a muscle.

There are a range of circumstances in which it is desirable to obtain an electrical measurement of a compound action potential (CAP) evoked on a neural pathway by an electrical stimulus applied to the neural pathway. However, this can be a difficult task as an observed CAP signal will typically have a maximum amplitude of a few tens of microvolts or less, whereas a stimulus applied to evoke the CAP is typically several volts. Electrode artefact usually results from the stimulus, and manifests as a decaying output of several millivolts or hundreds of microvolts throughout the time that the CAP occurs, presenting a significant obstacle to isolating the much smaller CAP of interest. As the neural response can be contemporaneous with the stimulus and/or the stimulus artefact, CAP measurements present a difficult challenge of implant design. In practice, many non-ideal aspects of a circuit lead to artefact, and as these mostly have a decaying exponential characteristic which can be of either positive or negative polarity, identification and elimination of sources of artefact can be laborious. A number of approaches have been proposed for recording a CAP, including those of King (US Patent No. 5,913,882), Nygard (US Patent No. 5,785,651), Daly (US Patent Application No. 2007/0225767) and the present Applicant (US Patent No. 9,386,934). US 2007/0225765 discloses a technique for adjusting the locus of excitation of electrically excitable tissue with paired pulses.

Evoked responses are less difficult to detect when they appear later in time than the artefact, or when the signal-to-noise ratio is sufficiently high. The artefact is often restricted to a time of 1 - 2 ms after the stimulus and so, provided the neural response is detected after this time window, data can be obtained. This is the case in surgical monitoring where there are large distances between the stimulating and recording electrodes so that the neural response propagation time from the stimulus site to the recording electrodes exceeds 2 ms. However, to characterize responses evoked by a single implant such as responses from the dorsal columns to SCS, for example, high stimulation currents and close proximity between electrodes are required, and therefore the measurement process must overcome contemporaneous artefact directly, greatly exacerbating the difficulty of neural measurement.

Similar considerations can arise in deep brain stimulation where it can be desirable to stimulate a neural structure and immediately measure the evoked compound action potential produced in that structure before the neural response propagates elsewhere in the brain. Artefact remains a significant obstacle to measurement of neural responses proximal to the stimulus location, with the consequence that most if not all conventional neurostimulation implants, which are necessarily compact devices, do not take any measurements whatsoever of neural responses evoked by the implant's stimuli.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed before the priority date of each claim of this application.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

In this specification, a statement that an element may be "at least one of" a list of options is to be understood that the element may be any one of the listed options, or may be any combination of two or more of the listed options.

### Summary of the Invention

The invention is defined in the appended claims. Aspects, embodiments and examples disclosed herein which do not fall within the scope of the appended claims do not form part of the invention, and are merely provided for illustrative purposes.

According to a first aspect the present invention provides a neurostimulation device comprising:
at least three stimulation electrodes configured to deliver an electrical stimulus to neural tissue; and
a control unit configured to deliver a first stimulus phase in which a first stimulus electrode delivers a supra-threshold stimulus component, returned by at least two other of the stimulation electrodes, the control unit further configured to deliver at least a second stimulus phase in which at least two of the stimulus electrodes deliver a sub-threshold stimulus component, returned by the first stimulus electrode.

According to an example which is not covered by the subject matter of the claims, there is provided a method of neurostimulation, the method comprising:
delivering an electrical stimulus to neural tissue using at least three stimulation electrodes, the electrical stimulus comprising a first stimulus phase in which a first stimulus electrode delivers a supra-threshold stimulus component returned by at least two other of the stimulation electrodes, and the electrical stimulus further comprising at least a second stimulus phase in which at least two of the stimulus electrodes deliver a sub-threshold stimulus component, returned by the first stimulus electrode.

According to another example which is not covered by the subject matter of the claims, there is provided a computer program product comprising computer program code means to make a neurostimulator execute a procedure for neurostimulation, the computer program product comprising computer program code means for carrying out the method of the second aspect.

Typically, the supra-threshold stimulus component will be a cathodic stimulus phase within the conventional meaning given to this nomenclature in the field of neurostimulation. In many neurostimulation applications, a cathodic stimulation threshold is lower than an anodic stimulation threshold so that a cathodic stimulus at a given amplitude may evoke a response where an anodic stimulus of equal amplitude and duration does not. However, the present invention is not to be understood as being limited by this theory and it is to be understood that in some embodiments the supra-threshold stimulus component may be an anodic stimulus phase, for example should it be the case that a certain neurostimulation application operates in an environment having a lower anodic stimulus threshold than cathodic stimulus threshold, or even should it be the case that alternative nomenclature is applied to the terms anode and cathode in particular stimulus modes or fields. Accordingly, references herein to a supra-threshold cathodic stimulus component are to be understood as being applicable to an anodic supra-threshold stimulus component in such alternative embodiments if applicable.

Preferred embodiments of the invention comprise at least three phases, delivered by at least three electrodes, and configured so that only one stimulus component from one electrode in one of the phases is a supra-threshold cathodic stimulus component. Such embodiments recognise that greater opportunities exist to maintain all of the stimulus components other than the supra-threshold cathodic stimulus component below a stimulus threshold, when three or more stimulus phases are utilised. For example, return current stimulus components, and charge balancing stimulus components, can be spread over a greater number of phases in such embodiments and thus may take reduced amplitudes in each such phase

The supra-threshold cathodic stimulus component is preferably delivered in a final phase of the stimulus, such as during the second phase of a biphasic stimulus. Such embodiments provide for greatest spatiotemporal separation of the stimulus from an onset of the ECAP, thus permitting ECAP measurement to occur sooner without saturation by the stimulus or stimulus artefact. In such embodiments it is to be noted that the first phase partial depolarisation may alter membrane potential and affect recruitment by the cathodic stimulus component in the second phase, however this may be resolved by suitable stimulus amplitude control. However alternative embodiments within the scope of the present invention may deliver the supra-threshold cathodic stimulus in a first phase, a penultimate phase or other non final phase of the stimulus. For example for triphasic stimuli the supra-threshold cathodic stimulus component is typically delivered in a second phase of the three phases.

Some embodiments may utilise three stimulus electrodes to deliver tripolar stimulation. Other embodiments may utilise four stimulus electrodes to deliver quadrupolar stimulation. For example offset quadrupolar stimulation may be delivered whereby the supra-threshold stimulus component is delivered by one electrode and returned by three electrodes. Other embodiments may utilise five or more stimulus electrodes to deliver pentapolar stimulation or to deliver stimuli from a greater number of electrode poles. Some embodiments may adaptively select the number of stimulus electrodes in order to seek which of a tripolar, quadrupolar, pentapolar etc configuration best minimises second cathode stimulation, by switching between such stimulation modes.

The current borne by the first stimulus electrode may in some embodiments be divided equally between the two or more other stimulus electrodes. For example in the case of tripolar stimulation having two return electrodes each return electrode may carry 50% of the current delivered by the first stimulus electrode. Alternatively, the current borne by the first stimulus electrode may be divided unequally between the two or more other stimulus electrodes while maintaining each at a sub-threshold level, and for example such unequal currents may be configured to take an inequality which minimises stimulus artefact in accordance with the teachings of the present Applicant's International Patent Application Publication No. WO 2017/219096, the content of which is incorporated herein by reference.

The current carried by each respective stimulus electrode may be controlled by providing a respective current source for each electrode configured to drive the desired current through that respective electrode in each phase of the stimulus. Alternatively, a respective current source may be provided between adjacent pairs of stimulus electrodes to effect differential drive of the desired current through each electrode. Such embodiments may be particularly applicable when unequal current sharing between the stimulus electrodes is required. Alternatively, one or more of the stimulus electrodes may be grounded to serve as a passive return electrode for example.

The first stimulus electrode in some embodiments is interposed between the two or more other electrodes, for example upon an epidural lead array.

In alternative embodiments the first stimulus electrode may be positioned to one side of both or all of the return electrodes. Such embodiments recognise that it is desirable to record an ECAP as close as possible to the site of stimulation, before ECAP propagation effects of a compound response cause dispersion and amplitude reduction of the initial neural response. In such embodiments, the return electrode stimulus components may be maintained at an equal level or at least at a respective sub-threshold level by driving each return electrode with a respective current source

Embodiments of the invention may further comprise recording electrodes and measurement circuitry, configured to obtain one or more recordings of a neural response evoked by the stimulus. In some such embodiments, the one or more recordings of the neural response are assessed in order to identify whether second cathode stimulation is occurring. For example, second cathode stimulation detection may be effected by applying stimuli of constant amplitude but having a distinct interphase gap(s), observing the ECAPs evoked by each such stimulus, and comparing the morphology of each such observed ECAP for indicia of second cathode stimulation. Additionally or alternatively, second cathode stimulation detection may be effected by applying stimuli of constant amplitude but having distinct stimulus phase sequencing, such as having alternating selection of phase delivery whether cathode first or anode first. Again in such embodiments the ECAPs evoked by each such stimulus may be observed and their morphology compared to detect any indicia of second cathode stimulation.

In embodiments where second cathode stimulation detection is provided, the outcome of such detection is preferably used to adjust the stimulus paradigm to seek prevention or reduction of second cathode stimulation when detected. Thus, preferred embodiments of the invention further provide for feedback control of the supra-threshold stimulus component, the feedback control configured to repeatedly or continuously assess one or more recordings of a compound action potential resulting from a preceding stimulus and to refine the stimulus including the supra-threshold stimulus component so as to seek to maintain neural recruitment from only the supra-threshold stimulus component.

Embodiments of the invention may utilise any suitable ECAP detector to assess the neural recordings to assess recruitment, such as an ECAP vector detector. The vector detector may for example utilise a four-lobed or five-lobed matched filter template in accordance with the teachings of the present applicant's International Patent Publication No. WO 2015/074121, the content of which is incorporated herein by reference. Particularly preferred embodiments may apply a stimulus in accordance with the present invention, detect an evoked response by using an ECAP vector detector, and use the output of the detector to control a stimulation feedback loop.

While safety requirements impose constraints on stimulus configuration it is to be noted that in some embodiments of the invention the stimulus itself might not be strictly charge balanced, and the net charge difference can be recovered by alternative means such as passively recovering charge by shorting one or more electrodes to ground at appropriate times.

Some embodiments of the invention may provide for automated stimulus amplitude determination, by occasionally applying stimuli at sub-threshold and supra-threshold amplitudes and observing evoked ECAP responses, determining therefrom a stimulus amplitude threshold at which ECAP responses are first evoked, and setting the ongoing therapeutic stimulus amplitude as a multiple of the stimulus amplitude threshold The multiplier may be determined at a time of device fitting on a patient by patient basis. Alternatively the multiplier may be selected to take a value in the range 1.05 to 1.8, more preferably in the range 1.1 to 1.4, more preferably in the range 1.15 to 1.25, more preferably 1.2 or about 1.2. Such embodiments recognise that clinical observations by the present applicant have revealed that a tripolar stimulus amplitude preferred by patients is often around 1.2 times the stimulus threshold for that patient.

### Brief Description of the Drawings

An example of the invention will now be described with reference to the accompanying drawings, in which:
Fig. 1 schematically illustrates an implanted spinal cord stimulator;
Fig. 2 is a block diagram of the implanted neurostimulator;
Fig. 3 is a schematic illustrating interaction of the implanted stimulator with a nerve;
Fig. 4A illustrates the profile of a prior art biphasic bipolar stimulus current over time, Figs. 4B and 4C illustrate delivery of each stimulus phase to a nerve, and Fig. 4D illustrates the respective electrode waveforms;
Figs. 5A and 5B illustrate an embodiment of the present invention utilising biphasic tripolar stimulation;
Fig. 6 illustrates some ECAP morphology parameters improved by the embodiment of Figs. 5A and 5B;
Fig. 7 illustrates an embodiment of the invention utilising a first arrangement of current sources to drive the tripolar stimulus electrode arrangement in accordance with one embodiment of the invention.
Fig. 8 illustrates another embodiment of the invention utilising a second arrangement of current sources to drive the tripolar stimulus electrode arrangement in accordance with another embodiment of the invention
Fig. 9 illustrates another embodiment of the invention utilising yet another arrangement of current sources to drive the tripolar stimulus electrode arrangement in accordance with yet another embodiment of the invention
Figs. 10A and 10B illustrate misalignment of the electrodes with the axis of propagation of the action potential.
Fig. 11A illustrates the area of polarisation and the area of partial depolarisation of a nerve, caused by a first phase of a stimulus, and Fig. 11B illustrates the additional areas of polarisation resulting in a second stimulus phase;
Fig. 12 further illustrates the problem of non-linear ECAP growth caused by multiple stimulus sites and times in conventional biphasic bipolar stimulation
Figs. 13 illustrates a method to detect the existence of second cathode stimulation; and
Fig. 14 illustrates a further embodiment of the present invention which further provides for automated optimisation of stimulation sequences to remove second cathode effects;
Fig. 15 schematically illustrates a system for feedback controlled neurostimulation in accordance with an embodiment of the invention;
Fig. 16 illustrates a triphasic tripolar stimulus configuration in accordance with another embodiment of the invention;
Figs. 17A and 17B illustrate the polarities of electrodes in a single phase of a quadrupolar stimulus configuration, and a pentapolar stimulus configuration, respectively, in accordance with further embodiments of the invention; and
Fig. 18 illustrates a triphasic quadrupolar stimulus configuration in accordance with yet another embodiment of the invention.

### Description of the Preferred Embodiments

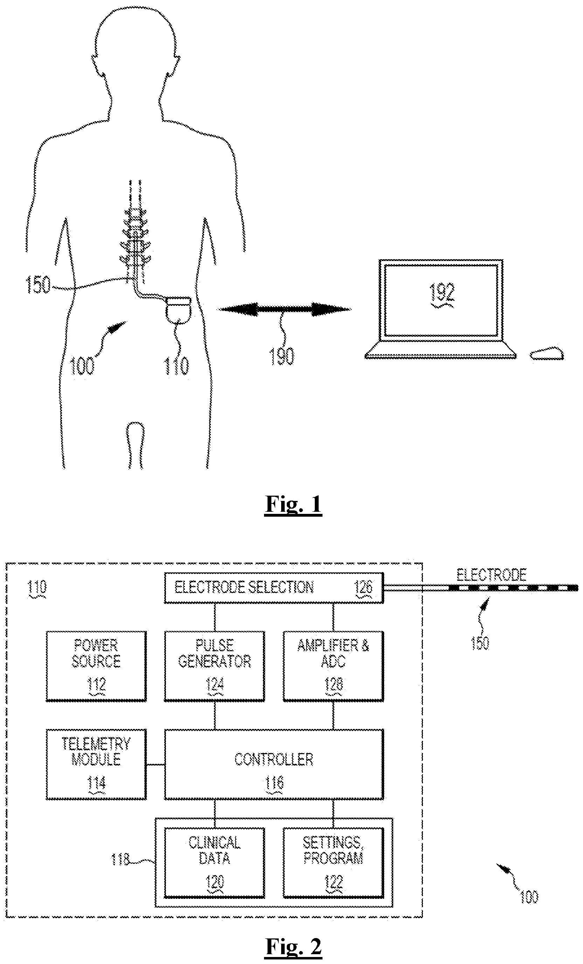

**Fig. 1** schematically illustrates an implanted spinal cord stimulator 100. Stimulator 100 comprises an electronics module 110 implanted at a suitable location in the patient's lower abdominal area or posterior superior gluteal region, and an electrode assembly 150 implanted within the epidural space and connected to the module 110 by a suitable lead Numerous aspects of operation of implanted neural device 100 are reconfigurable by an external control device 192. Moreover, implanted neural device 100 serves a data gathering role, with gathered data being communicated to external device 192 via any suitable transcutaneous communications channel 190.

**Fig. 2** is a block diagram of the implanted neurostimulator 100. Module 110 contains a battery 112 and a telemetry module 114. In embodiments of the present invention, any suitable type of transcutaneous communication 190, such as infrared (IR), electromagnetic, capacitive and inductive transfer, may be used by telemetry module 114 to transfer power and/or data between an external device 192 and the electronics module 110. Module controller 116 has an associated memory 118 storing patient settings 120, control programs 122 and the like. Controller 116 controls a pulse generator 124 to generate stimuli in the form of current pulses in accordance with the patient settings and control programs 122. Electrode selection module 126 switches the generated pulses to the appropriate electrode(s) of electrode array 150, for delivery of the current pulse to the tissue surrounding the selected electrode(s). Measurement circuitry 128 is configured to capture measurements of neural responses sensed at sense electrode(s) of the electrode array as selected by electrode selection module 126.

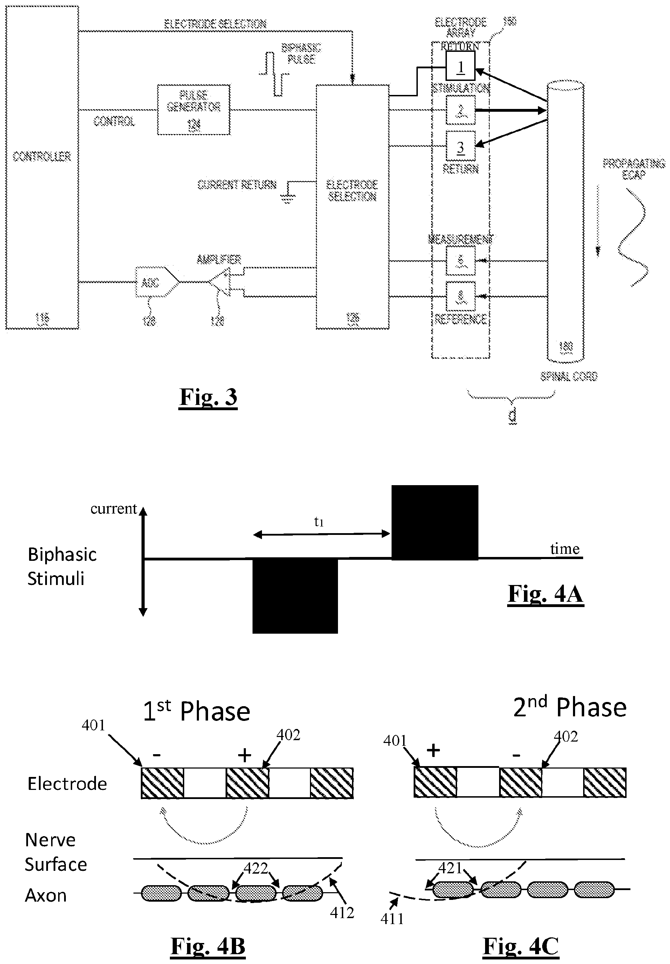

**Fig. 3** is a schematic illustrating interaction of the implanted stimulator 100 with a nerve 180, in this case the spinal cord however alternative embodiments may be positioned adjacent any desired neural tissue including a peripheral nerve, visceral nerve, parasympathetic nerve or a brain structure. Electrode selection module 126 selects a stimulation electrode 2 of electrode array 150 to deliver an electrical current pulse to surrounding tissue including nerve 180, and also selects two return electrodes 1 and 3 of the array 150 for stimulus current recovery to maintain a zero net charge transfer.

Delivery of an appropriate stimulus to the nerve 180 evokes a neural response comprising a compound action potential which will propagate along the nerve 180 as illustrated, for therapeutic purposes which in the case of a spinal cord stimulator for chronic pain might be to create paraesthesia at a desired location. To this end the stimulus electrodes are used to deliver stimuli at any therapeutically suitable frequency, for example 30 Hz, although other frequencies may be used including as high as the kHz range, and/or stimuli may be delivered in a non-periodic manner such as in bursts, or sporadically, as appropriate for the patient. To fit the device, a clinician applies stimuli of various configurations which seek to produce a sensation that is experienced by the user as a paraesthesia. When a stimulus configuration is found which evokes paraesthesia, which is in a location and of a size which is congruent with the area of the user's body affected by pain, the clinician nominates that configuration for ongoing use.

The device 100 is further configured to sense the existence and intensity of compound action potentials (CAPs) propagating along nerve 180, whether such CAPs are evoked by the stimulus from electrodes 1-3, or otherwise evoked. To this end, any electrodes of the array 150 may be selected by the electrode selection module 126 to serve as measurement electrode 6 and measurement reference electrode 8. Signals sensed by the measurement electrodes 6 and 8 are passed to measurement circuitry 128, which for example may operate in accordance with the teachings of International Patent Application Publication No. WO2012155183 by the present applicant, the content of which is incorporated herein by reference. The output of circuitry 128 is used by controller 116 in a feedback arrangement to control the application of subsequent stimuli, and the controller 116 also stores the recording of the neural response or one or more parameters thereof such as ECAP amplitude to the Clinical Data storage 120.

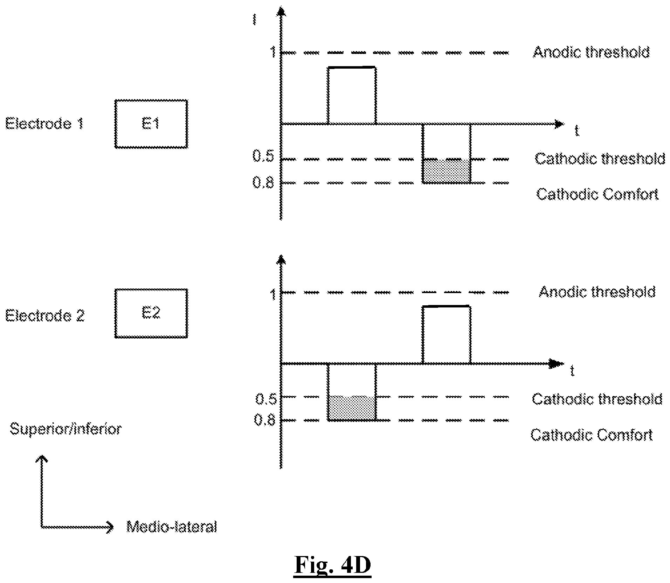

To ensure patient safety it is necessary to ensure that stimuli are delivered in a charge balanced manner, and moreover that a bidirectional current is passed through each stimulus electrode to counteract electrochemical effects which unavoidably arise at the electrode-tissue interface. This imposes certain limitations on the allowable configurations of stimuli which may be used in any practical neurostimulation device. A conventional stimulus used in such devices is a biphasic bipolar stimulus as depicted in Fig. 4A - 4D. Fig. 4A illustrates the profile of the stimulus current over time. Figs. 4B and 4C illustrate delivery of each respective stimulus phase to a nerve. Fig. 4D illustrates that a biphasic bipolar stimulus unavoidably delivers at least two supra-threshold stimulus components.

In this conventional biphasic bipolar stimulus arrangement, a first stimulus phase delivers a current from electrode 402, and the current is returned by electrode 401. In a second phase an equal and opposite current is delivered, that is, a current is delivered from electrode 401 and returned by electrode 402. The stimulus is thus biphasic as it delivers two phases, and is bipolar as it utilises two electrodes. As shown in Fig. 4D, for effective therapy the stimulus amplitude must be set to a cathodic comfort level. The cathodic comfort level is usually of lesser magnitude than an anodic threshold level, meaning that only the cathodic stimulus component of each phase gives rise to neural recruitment.

However, due to the aforementioned safety constraints, the cathodic excitation of electrode 402 in the first phase (Fig. 4B) is equal to the cathodic excitation of electrode 401 in the second phase (Fig. 4C), but at times t₁ apart. At a first time, in phase 1 (Fig. 4B), the cathodic excitation of electrode 402 evokes a neural response only on the Node(s) of Ranvier 422 which are triggered by electrode 402, depicted for illustrative purposes by excitation range 412. At a second time, t₁ later than the first time, in the second phase (Fig. 4C) the cathodic excitation of electrode 401 evokes a neural response only on the Node(s) of Ranvier 421 which are triggered by electrode 401, depicted for illustrative purposes by excitation range 411. It is to be noted that excitation range 412 is adjacent electrode 402, and is not coterminous with excitation range 411 which is adjacent electrode 401. Thus, the stimulation site in the first phase is at least partly different, or even entirely different, to the stimulation site in the second phase of the stimulus.

While for typical values of t₁ a given axon recruited by the first phase will typically be refractory during the second phase and will not be able to be recruited again, other axons (not shown in Figs. 4A - 4D) not recruited by the first phase remain available for excitation by the second phase in the manner described. The respective sub-population of fibres available for recruitment in the second phase is unknown or at least very poorly known, introducing considerable uncertainty about the respective amplitude of compound response which might arise in the second phase.

Accordingly, biphasic bipolar stimulation typically evokes a compound response made up of neural response components which arise at different times, and which arise at different locations, and which are of unequal amplitude with the extent of amplitude inequality being unknown. These neural response components overlap in, and jointly contribute to, electrical observations of the neural response as a whole, significantly impeding attempts to accurately understand the actual recruitment effect of each component of the stimulus

In contrast to the conventional bipolar biphasic stimulation shown in Figs. 4A - 4D, the present invention proposes a tripolar (or greater) stimulus, configured in a particular manner to seek a paradigm in which only one component of the stimulus recruits any neural response. When achieved, this outcome means that the resulting compound action potential can be tied to a particular single stimulus site, and to a particular single stimulus time, for example for the purposes set out in the present Applicant's International Patent Publication No. WO 2016/161484, the content of which is incorporated herein by reference. Determinations of conduction velocity can also be improved, both because the stimulus site and time is known with improved accuracy and because the observed response is less time-spread making for sharper peaks and thus allowing improved resolution of the time of occurrence of such peaks when assessing time of arrival for conduction velocity determinations.

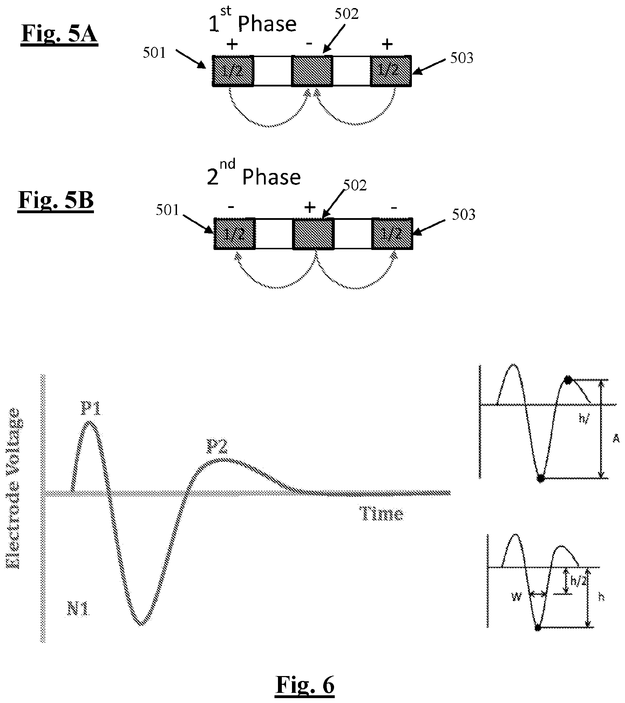

**Figs 5A and 5B** illustrates a biphasic tripolar stimulation. In the first phase shown in Fig. 5A, stimulus electrodes 501 and 503 are cathodic and deliver current to the tissue, with all current returned by the central anodic stimulus electrode 502. For illustrative purposes, electrodes 501 and 503 deliver a nominal 0.5 units of current, while electrode 502 returns 1 unit of current. In the second phase shown in Fig. 5B, electrode 502 delivers 1 unit of current, and electrodes 501 and 503 each return 0.5 units of current. In accordance with the present invention, the current amplitude delivered is carefully selected to ensure that the cathodic stimulation threshold is in the range 0.5 - 1 units of current, and to ensure that the anodic stimulation threshold is greater than 1 unit of current. This arrangement thus provides for recruitment to be caused by a single cathode flanked by anodes on either side. Consequently, only one phase of this stimulus paradigm (the second phase), and only one electrode (electrode 502) ever gives rise to any neural recruitment, thus ensuring that the time and location of stimulation can be known with the greatest possible accuracy. For example this can allow the ECAP morphology to be assessed with confidence that a second stimulus site and time is not contributing to the ECAP.

In particular, the anodic current of electrode 502 in phase 1, and of electrodes 501 and 503 in phase 2, can be ignored at the stimulation levels of typical neurostimulation practice. This is because the typical stimulation levels required for effective therapy are between the electrophysiological threshold (the current at which it is first possible to detect ECAPs), and about two times that threshold current. While anodic recruitment is possible, the threshold current for action potential generation by the anode is significantly higher, with the threshold current for anodic excitation typically being about 5 to 8 times higher than for cathodic excitation. The present invention recognises that this presents a useful range of operation within which it is possible to maintain a single supra-threshold stimulus component. In embodiments using tripolar stimulation for this purpose, this ensures that the additional cathodes generated on the flanking electrodes (501, 503) in the additional phase is below the threshold for stimulation when the stimulation current is maintained below 2 times threshold. This particular configuration of the stimulus thus ensures that action potentials are generated from a single cathode in, i.e. by a single supra-threshold stimulus component of, the overall biphasic tripolar stimulus. We aim to measure the ECAP as close to the stimulus as possible, and as soon after the stimulus as possible, and so the stimulus is presented with an anode on the centre electrode of the tripole in the first phase, so that the cathodic stimulus component can be presented in the second and final phase of the stimulus allowing measurements to be commenced immediately thereafter.

ECAP morphology parameters which might for example be assessed include those shown in **Fig. 6****.** A single action potential comprises a P1 peak indicating initial depolarization (cell capacitance) followed by an N1 valley or negative peak indicating depolarization (sodium channel activation) and finally a P2 peak indicating hyperpolarization (potassium channel activation). ECAP morphology parameters which might for example be assessed include characteristics such as P1-N1 peak-to-peak amplitude, N1-P2 peak-to-peak amplitude, P1 peak time, P1 peak amplitude, P1 half height peak width, P1-N1 zero crossing time, P1-N1 zero crossing slope, N1 peak time, N1 peak amplitude, N1 half height peak width, N1-P2 zero crossing time, N1-P2 zero crossing slope, P2 peak time, P2 peak amplitude, P2 half height peak width, and the like. As all of these parameters can respond non-linearly to stimulus amplitude if the stimulus recruits neurons at multiple different sites and at multiple different times, embodiments of the present invention may significantly improve the reliability of any or all such ECAP morphology parameters when used as feedback variables or as diagnostic indicators or the like.

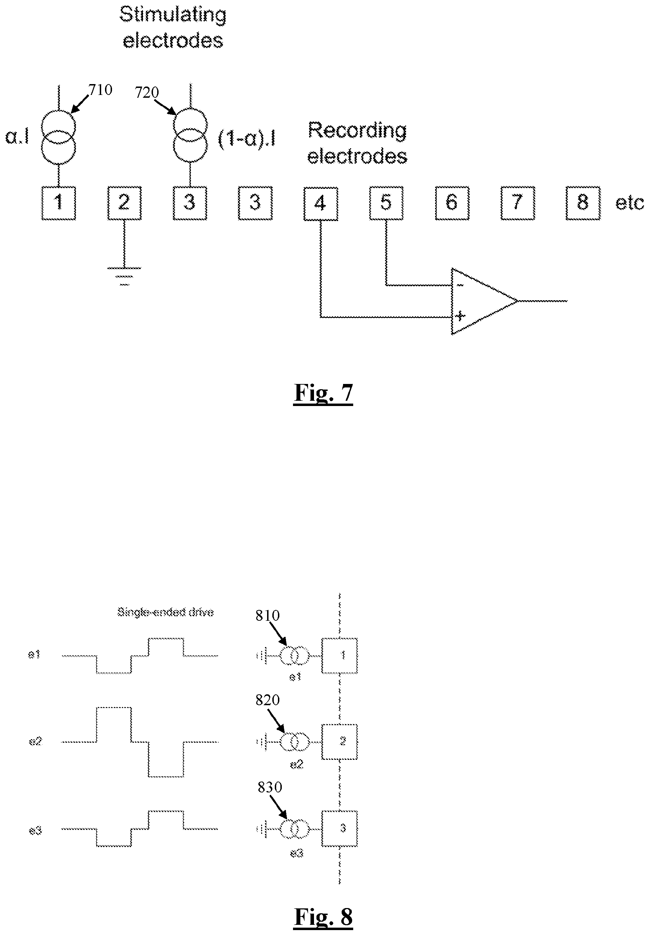

**Fig. 7** illustrates a first arrangement of current sources to drive the tripolar stimulus electrode arrangement in accordance with one embodiment of the invention. In this embodiment, two current sources 710 and 720 are used to effect unequal current sharing between electrodes 1 and 3. Electrode 2 is simply grounded to provide a return path for the entire current delivered by the current sources.

**Fig. 8** illustrates a second arrangement of current sources to drive the tripolar stimulus electrode arrangement in accordance with another embodiment of the invention. In this embodiment, each of electrode 1, electrode 2 and electrode 3 are provided with a respective single ended current source 810, 820, 830. The current sources are configured to deliver respective individual single-ended drive current profiles denoted e1, e2 and e3.

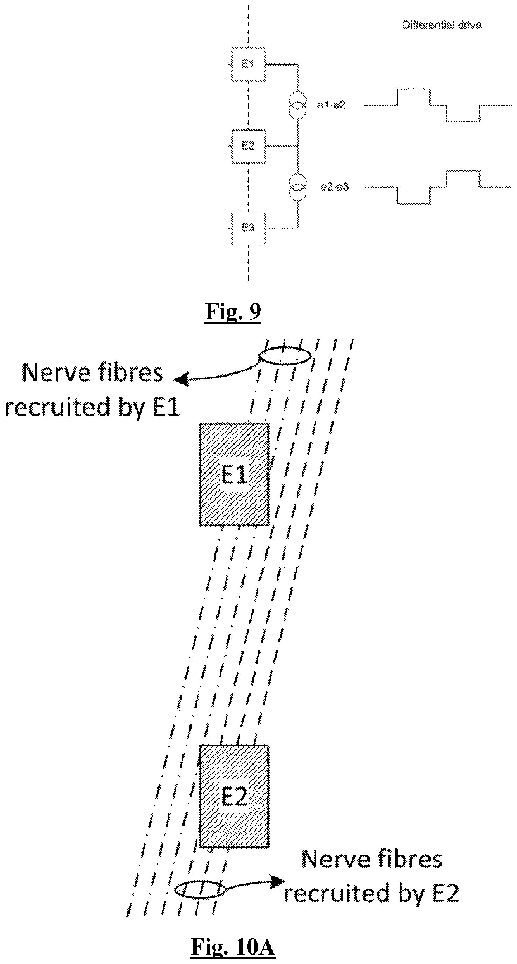

**Fig. 9** illustrates yet another arrangement of current sources to drive the tripolar stimulus electrode arrangement in accordance with yet another embodiment of the invention. This is a differential drive arrangement equivalent to the single ended drive arrangement of Fig. 8.

Thus, use of three electrodes to effect tripolar stimulation in this manner in the present embodiment means that in the first phase of the biphasic stimulus the stimulus current is delivered from both the electrodes 1 and 3 (Figs. 3, 7, 8, 9) which in this first phase are cathodic, and the entire return current is carried by electrode 2 which is anodic. And, in the second phase of the biphasic stimulus, the entire stimulus current is delivered from electrode 2 which in this phase is cathodic, while the return current is shared between the return electrodes 1 and 3 which in this phase are anodic. Thus, in both the first phase and the second phase of the stimulus, the sum of the current through electrode 1 (I₁) and the current through electrode 3 (I₃) is equal and opposite to the current through electrode 2 (I₂). That is, I₁ + I₃ = -I₂.

The present invention recognises that it is possible to ensure that only one of these stimulus components evokes a neural response, being the cathodic current on electrode 2. This is because it is possible to arrange the tripolar stimulus so that the cathodic currents on electrodes 1 and 3 in phase 1 never exceed about half the cathodic current delivered in phase 2 by electrode 2. It is therefore possible to maintain the cathodic current on electrodes 1 and 3 in the first phase below a stimulus threshold, while maintaining the cathodic current on electrode 2 in the second phase above the stimulus threshold. And, while the anodic current on electrode 2 in the first phase must for safety reasons typically be substantially equal (but opposite to) the cathodic current on electrode 2 in the second phase, the anodic stimulus threshold is typically greater than the cathodic stimulus threshold, so that the anodic current on electrode 2 in the first phase may be kept below an anodic stimulus threshold while maintaining the (equal but opposite) cathodic current on electrode 2 in the second phase above the cathodic stimulus threshold.

Thus, the biphasic tripolar stimulus may be configured to satisfy the requirements for charge balancing, and further to satisfy the requirement for all electrochemical effects at the electrode-tissue interface to be reversed by the use of alternating currents at each electrode, while nevertheless delivering a stimulus in which only one component of the stimulus evokes a neural response.

Further benefits and embodiments of the invention are also envisaged. In this regard, it is noted that one field of application for some embodiments of the invention is spinal cord stimulation therapy, which achieves its therapeutic benefit via stimulation of the dorsal columns of the spinal cord with electrodes that are placed in the epidural space. The dorsal column is arranged such that layers within the column radiating away from the midline carry nerve fibres which innervate a particular region (or dermatome) of the body. The goal of electrical spinal cord stimulation therapy is to recruit those fibres which correspond to the dermatomes where pain is present. Stimulation in adjacent areas where no pain is present can be uncomfortable for the recipient of the device, so much so that stimulation in unwanted areas can lead to recipients discontinuing their therapy.

There is considerable variation in the current required to achieve consistent recruitment of dorsal column fibres, and feedback loops have been established to maintain recruitment at a constant desired level. One technique relies on the use of the electrically evoked compound action potential as a measure of the spinal cord excitation, as described in the present Applicant's International Patent Publication No. WO 2012/155188, the content of which is incorporated herein by reference. When considering the use of the ECAP as an aid in establishing programing parameters which can be used to maintain consistent recruitment of the desired dermatomal areas to the extent possible, the present invention recognises that ECAPs evoked at multiple sites and/or at multiple different times by conventional biphasic bipolar stimuli can complicate the task of interpreting an observed ECAP and thus impede effective therapy.

A cathodic current has a higher recruitment efficiency than an anodic current and so it is desirable to employ cathodic currents, however monophasic pulses are unsafe and so the cathodic current must be accompanied shortly after with an anodic current to reverse the potential. This has the effect of reversing any electro chemistry which has taken place on the electrode surface. The duration and amplitude of this balancing pulse can be different to the duration and amplitude of the cathodic pulse (but of opposite sign) provided that the charge delivered during the two phases (duration * amplitude) is identical.

The simplest form of charge reversal is the use of biphasic stimuli pulse. The spread of the electric field from the stimulus is governed by the separation between the electrodes which for SCS devices are usually electrodes on the same lead. The stimulation occurs with either the cathode first or the anode as illustrated in Fig. 4. In the illustration the cathodic and anodic phases have the same pulse width but are opposite sign and this results in current flowing (from positive, in line with convention) to negative in the first phase and then the opposite in the second phase with the position of the anode and cathode swapped.

The net result of this is that for every single biphasic stimulus there arises two cathodes, one from the first phase and one from the second phase. In an ideal situation, with the cathode first, the stimulus recruits the fibres which then enter their refractory period and remain so during the presentation of the second phase. In this ideal situation no recruitment of any additional fibres occurs and the stimulus location is mapped to a single electrode.

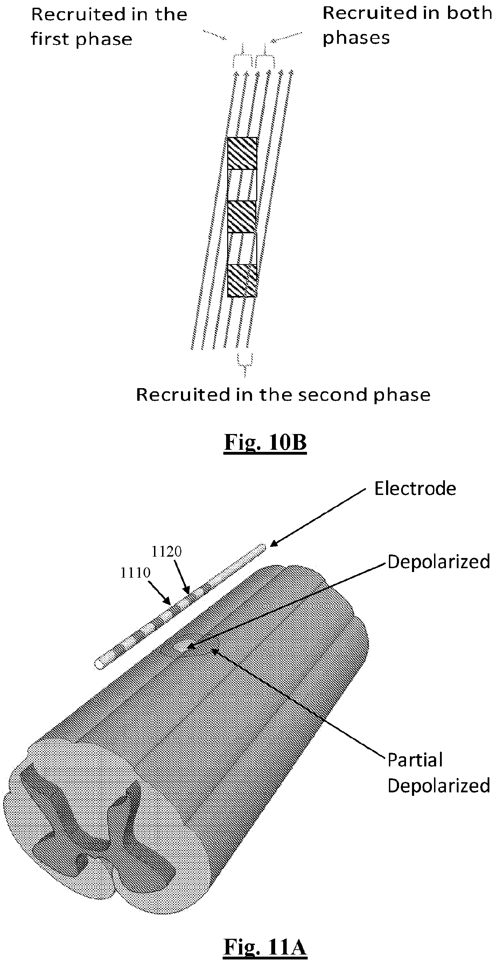

However the present invention recognises that in practice this situation is impossible to achieve. In addition to the reasons discussed in the preceding, it is important to note the possibility of misalignment of the electrodes with the axis of propagation of the action potential, as illustrated in **Figs. 10A** **and** **10B****.** Such misalignment can result from surgical misalignment of an electrode lead adjacent a nerve, and/or due to individual nerve fibres taking a somewhat wandering path within the nerve as is known to occur. In the event of such misalignment of the electrodes with the axis of propagation of the action potential, the first cathode can produce a partly depolarised membrane but fail to initiate an action potential, which the second cathode may then initiate if the second cathode is presented is within a short enough window. This geometric limit is illustrated in Figs. 10A and 10B. The first phase of stimulation recruits a group of fibres and the second phase recruits some additional fibres not recruited by the first phase of stimulation. When the fibres recruited in the two phases correspond to different dermatomal areas then this would result in perception of stimulation in different locations and can lead to stimulation in undesired locations. Indeed, in the case of Fig. 10B such misalignment can even lead to dermatomes on the wrong side of the body being recruited, for example where stimulation on the right side is desired, such geometric misalignment can inappropriately increase the chance of stimulation incorrectly occurring on the left side of the body.

There is not only a geometric constraint to maintenance of recruitment to single location but there is also a timing constraint. The stimulus current not only depolarises a region of the nerve to produce action potentials but, due to the nature of the electric field, partly depolarises a more extended area around the area which has been generated action potentials. This is illustrated in **Fig. 11A****.** Bipolar stimulation is commonly used during SCS and the 1st phase anode 1120 is often the adjacent electrode to the cathodic stimulus electrode 1110. As shown in Fig. 11A, the first phase cathodic stimulus delivered by electrode 1110 creates an area upon the nerve in which axons will depolarise and initiate action potentials. In an annular region around this area is a second area in which the excitation is sufficient to cause partial depolarisation of membranes to a degree which does not cause action potentials but which does lead to altered membrane conditions at a later time when the second phase is applied.

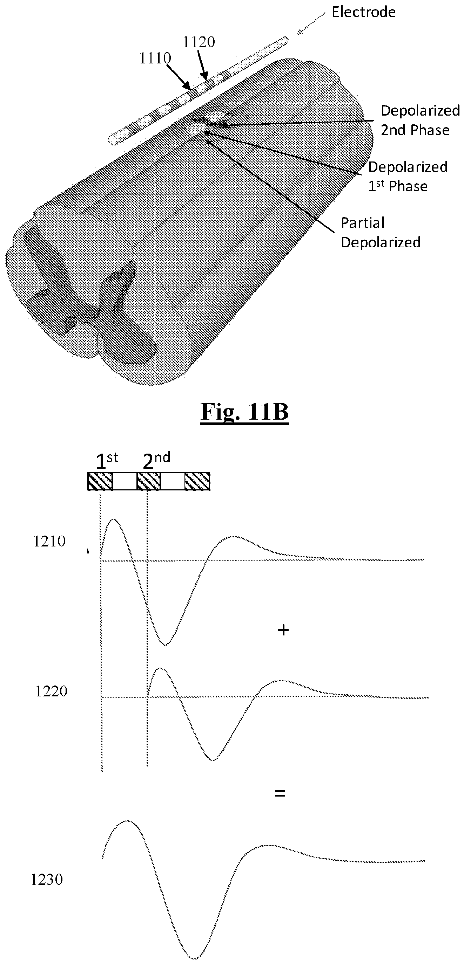

**Fig. 11B** illustrates the areas of the nerve excited by a second phase. Under these circumstances, even if the geometric alignment of the electrode array and the nerve is precise and the field from the 2nd cathode is applied only to fibres recruited by the first phase, the second phase also produces a second zone of partial depolarisation, and the overlap of this area with the area of partial depolarisation produced by the first phase would generate additional action potentials, thus giving rise to another undesirable mechanism by which the time and location of recruitment is confused due to the configuration of the applied stimulus.

The situation is further complicated when other confounding factors are considered. For instance the stimulation threshold for the two different locations for the first cathode 1110 and second cathode 1120 may be different from each other. In a situation where the threshold for activation is higher under the second cathode 1120 then the strength of stimulation in the undesired corresponding dermatomal location would be stronger. In the most extreme case where the threshold for the first cathode 1110 is highest of all the electrodes available, but it is the electrode best placed for use corresponding to the painful dermatome, stimulation of the painful area can never be achieved with biphasic stimuli without stimulation in unwanted areas by electrode 1120 and this leads to either non acceptance of the therapy or a diminution in performance.

The variation in recruitment which occurs with postural changes further confounds the goal of targeting the stimulation. It is desirable to produce a device which achieves closed loop control of the ECAP amplitude to provide recipients with additional benefit not the least of which is elimination of changes in therapy as a result of postural effects. Ideally the ECAP amplitude would maintain the same shape but grow in amplitude with increasing stimulation current with the form of k(I-T)x(t) where T is threshold, k is a gain term. That is, in this ideal case the shape of the ECAP scales linearly with the current. This would provide a key benefit of allowing simple detector designs one of which may consist of simply computing the dot product of the signal with an appropriately designed filter function as described in the present applicant's WO 2015/074121. In the event that any stimulus design produces recruitment in two locations and at two different times then any small change in threshold between the two locations would produce an ECAP shape which changes with increasing current, voiding the assumption of linear ECAP amplitude response and significantly complicating the task of assessing recruitment from detected ECAPs.

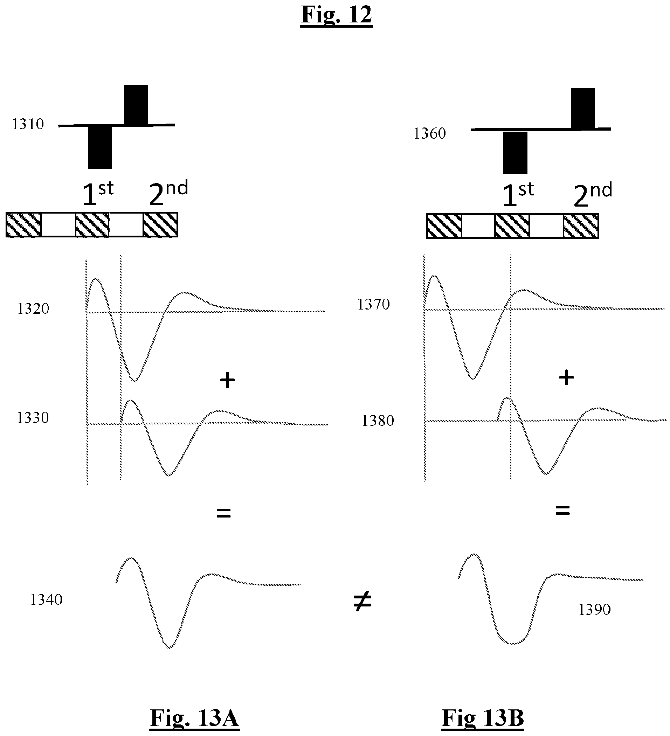

**Fig. 12** further illustrates the problem of non-linear ECAP growth caused by multiple stimulus sites and times in conventional biphasic bipolar stimulation. Between threshold for the first cathode and threshold for the 2nd cathode a first ECAP shape A is present. Above the threshold for the 2nd cathode another ECAP shape B emerges. For certain combinations of distance between electrodes and fibre population conduction velocity the negative and positive peaks of the ECAPs can align with each other cancelling each other. This in turn creates conditions where increasing stimulation current counterintuitively produces decreasing ECAP amplitude and this leads to feedback loop instability during closed loop control. It is thus highly desirable to detect and eliminate any 2nd cathode effects, particularly for closed loop controlled neuromodulation because it allows simplified detectors and improves loop stability.

Because the two modes of stimulation (first cathode, second cathode) can have different thresholds, and the ECAPs initiate at different locations along the dorsal column, then the ECAP morphology can change with current. When stimulus current is increased from a sub-threshold level to where the first threshold is reached, ECAP shape A emerges. When, with further increase in stimulus current, the second threshold is reached, the second ECAP B emerges. ECAP B has the same shape as ECAP A but is offset in time from the first. The sum of A & B has a shape that is different and this compound response is what can be observed by recording electrodes and accompanying measurement circuitry and an ECAP detector module It is possible that the second ECAP causes the detector output to fall rather than rise, leading to loop instability or diminished performance.

These non-linear effects do not occur when recruitment only occurs at a single stimulus site at a single time, as occurs in accordance with embodiments of the present invention. Under such conditions an ECAP with constant shape emerges and grows linearly with stimulus amplitude as is desired to ease the difficult task of ECAP detection

To optimise a stimulation paradigm which is better targeted to the desired dermatome, some embodiments of the invention further take steps to actively identify whether second cathode stimulation is occurring, and to take steps to prevent second cathode stimulation when detected. To detect the occurrence of second cathode stimulation is not possible on the basis of patient feedback as the strength of perception grows as the current is increased in both desired and undesired areas and in a uniform manner, so that the patient cannot discern any useful distinctions to report on. However, these embodiments of the invention recognise that identification of second cathode affects can be achieved from measurements of the electrically evoked compound action potential.

The recorded potential is sum of potential produced by both the first and second cathode, as depicted in Fig. 12. The ECAP which is actually measured by recording electrodes some distance away from the stimulus electrodes is thus the sum of the responses 1210 and 1220 from both cathodes which are initiated at different times in the stimulus cycle. While the recording obtained from the recording electrodes can only ever reflect the compound response 1230 made up of components 1210 and 1220 together, the present invention recognises that there are a number of ways to detect if a second cathode stimulation is occurring.

A first manner in which to detect second cathode stimulation is to inspect the recorded response 1230 to identify whether there exists two observable N1 peaks, which may occur when the two cathodes are separated by a suitable distance. However, this approach is not able to identify a large range of scenarios in which second cathode stimulation occurs but does not manifest in two observable N1 peaks.

Accordingly, an alternative method to detect the existence of second cathode stimulation involves varying pulse parameters which do not affect recruitment but which will bring about a change in the observed compound action potential should any second cathode stimulation be taking place. One example, shown in **Fig. 13****,** involves varying the interphase gap. A first stimulus 1310 is applied, having a first interphase gap between the two phases of the stimulus. A first recording 1340 of the resulting ECAP is obtained. A second stimulus 1360 is applied at another time, having a second interphase gap different to the first interphase gap. Both the first interphase gap and the second interphase gap should be maintained within the absolute refractory period, which for example can be measured in accordance with the teachings of the present Applicant's International Patent Publication No. WO 2012/155189, the content of which is incorporated herein by reference. As shown in Fig 13A & Fig. 13 B, because each phase of the stimuli 1310 and 1360 are the same, the respective response components 1320 and 1370 are the same, and the respective response components 1330 and 1380 are the same. However, the time spacing between the respective response components differs, so that the compound responses 1340 and 1390 are different and can thus be inspected for indicia of second cathode stimulation. For example, comparing ECAP parameters such as one or more peak amplitudes, one or more peak to peak amplitudes, and/or one or more peak widths, allows the different morphology of responses 1340 and 1390 to be detected. A degree by which such metrics (or a combination thereof) differ can be taken as being indicative of a degree to which second cathode stimulation is occurring, which in turn could for example be used to control a feedback loop gain or to assist PID loop control to avoid overshoot to provide control over how rapidly the system responds to detected second cathode stimulation for example.

There are many alternative schemes for detecting the presence of second cathode stimulation in accordance with embodiments of the present invention. For instance, one such embodiment may utilise a set of stimulus electrodes, and deliver a first stimulus from one subset of the electrodes and measure a first neural response evoked by the first stimulus. A second stimulus may then be delivered from a different subset of the electrodes and a second neural response evoked by the second stimulus may be measured. Provided the positions of the stimulus electrodes relative to each other remains the same, and assuming the thresholds for cathodic stimulation are the same, then a comparison of the first and second neural responses would yield an indication of whether second cathode stimulation is present.

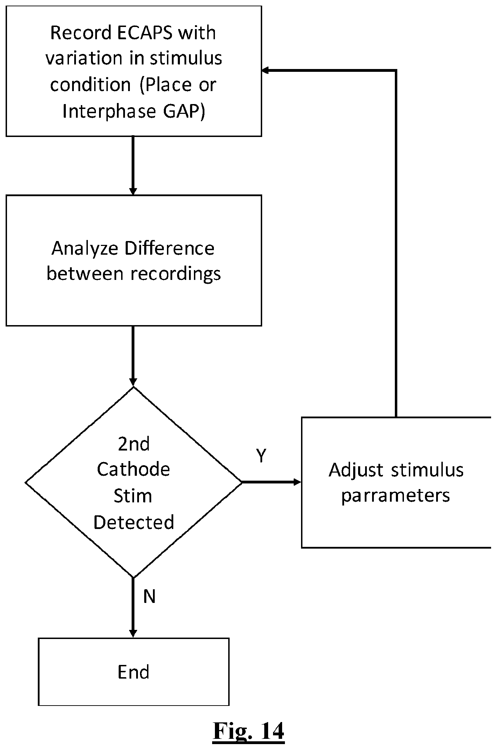

**Fig. 14** illustrates an automated optimisation of stimulation sequences to remove second cathode effects. This detection method provides a means to optimise the stimulation parameters to avoid the above noted undesirable effects of second cathode stimulation. It is to be noted that even when exploiting tripolar stimulation as taught for example in Fig. 5, it remains possible that the threshold for cathodic stimulation for electrodes 501 and/or 503 could feasibly be significantly lower than the cathodic stimulation threshold of electrode 502 and that the 0.5 units of cathodic excitation delivered from electrodes501 and 503 in phase 1 could in such instances give rise to second or even third cathode stimulation. Accordingly, even when utilising such a tripolar stimulation configuration, it remains desirable to monitor for any instances of second cathode stimulation. Accordingly, in the embodiment of Fig. 14 a process is implemented which automatically adjusts the tripolar stimulus based on the detection of any second cathode effects, to thereby iteratively adjust one or more stimulus parameters. For instance in the tripolar stimulus pattern, the threshold for stimulation can be increased (i.e. recruitment by electrodes 501 and 503 can be decreased and preferably eliminated) by extending the pulse width of the respective anodic phase and reducing the current amplitude, which is therapeutically acceptable provided that (1) the total time for both phases is less than the refractory period, and (2) the same charge is delivered in both phases. Within these constraints it is possible to tune the stimulus to prevent any form of second cathode stimulation.

Further parameters can be revised to seek the elimination of second cathode effects, including for instance increasing the number of anodes, increasing the number of stimulus electrodes from three to five so as to shift from tripolar stimulation to pentapolar stimulation, or using four stimulus electrodes with three return electrodes in an offset-quadrupolar arrangement, adjusting the sharing of current between electrodes to be unequal in cases where this improves the elimination of second cathode stimulation, increasing the number of stimulus phases to provide greater ability to keep sub-threshold components further below a respective stimulation threshold, and so on.

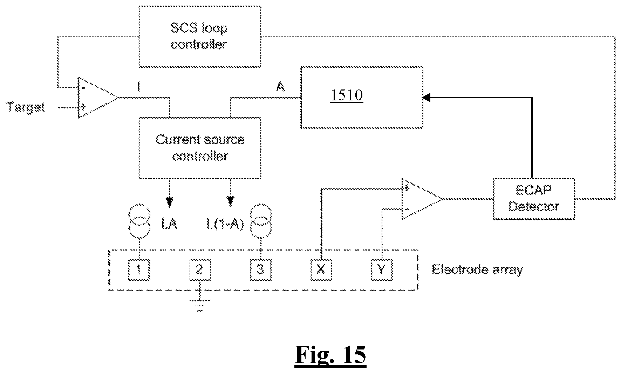

Fig. 15 schematically illustrates a system for feedback controlled neurostimulation utilising the above-described principles. In particular, control block 1510 is configured to control a current sharing ratio defined by parameter A, and is also configured to control multipolar mode selection and multiphasic mode selection in accordance with the principles set forth in the preceding.

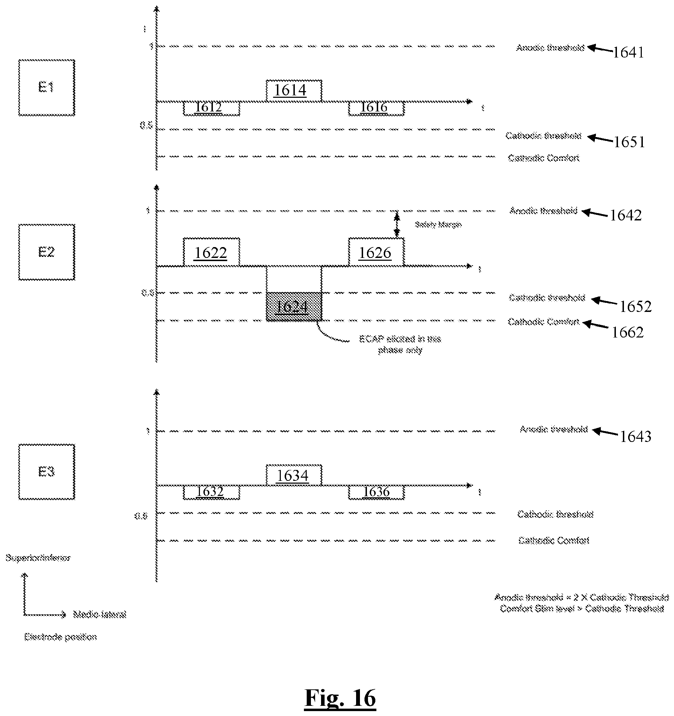

Fig. 16 illustrates a triphasic tripolar stimulus configuration. In this embodiment, the second phase delivered on electrode E2, denoted 1624, is configured to be the only cathodic stimulus component which elicits a neural response. This is because stimulus component 1624 is the only component which exceeds a stimulus threshold, namely cathodic stimulus threshold 1650. Stimulus component 1624 is set to an amplitude which elicits the desired therapeutic effect, referred to as the cathodic comfort level 1662.

All other components of the stimulus then can be derived from the amplitude of stimulus component 1624. Specifically, if we define the amplitude of stimulus component 1624 as X, then anodic stimulus components 1614 and 1634 delivered by electrodes E1 and E3 respectively should take an amplitude of 0.5 X to effect 100% current return in the second phase. Anodic stimulus components 1614 and 1634 can take alternative amplitudes (such as 0.7 and 0.3, respectively) provided the net anodic amplitude adds to X to effect 100% current return in the second phase.

Similarly, anodic stimulus components 1622 and 1626 delivered by electrode E2 in the first and third phases respectively should each take an amplitude of 0.5 X to effect current balancing of electrode E2 across the three phases. Anodic stimulus components 1622 and 1626 can take alternative amplitudes (such as 0.7 and 0.3, respectively) provided the net anodic amplitude of these components adds to X in order to effect current balancing of electrode E2 across the three phases.

Finally, cathodic stimulus components 1612 and 1632 delivered by electrodes E1 and E3 respectively in the first phase should each take a value of 0.25 X, or should at least sum to 0.5 X, to effect 100% current return in the first phase. Similarly, cathodic stimulus components 1616 and 1636 delivered by electrodes E1 and E3 respectively in the third phase should each take a value of 0.25 X, or these components should at least sum to 0.5 X, to effect 100% current return in the third phase. A further constraint is that current balancing of electrode E1 should be effected, and current balancing of electrode E3 should be effected, across the three phases, as can be simply achieved.

Fig. 16 thus provides for the cathodic stimulus component 1624 to be four times larger than any other cathodic stimulus component (1612, 1616, 1632, 1636), thus permitting significant variation of the amplitude of component 1624 for therapeutic purposes while retaining all other cathodic stimulus components below threshold. It is further noted that no anodic stimulus component delivered by any electrode exceeds the respective anodic stimulus threshold (1641, 1642, 1643). Thus, the stimulus configuration of Fig. 16 provides for single cathode neural excitation as desired.

In the example of Fig. 16 the anodic threshold is indicated as being double the magnitude of the cathodic threshold however it is to be appreciated that this relationship might differ in other embodiments within the scope of the present invention.

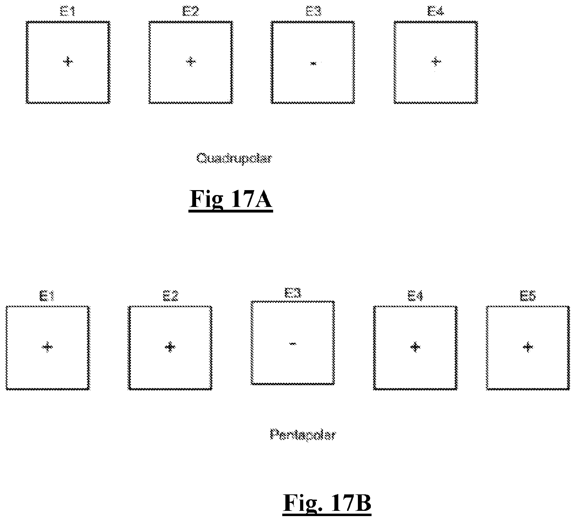

Fig. 17A illustrates the polarities of electrodes in a single phase of a quadrupolar stimulus configuration in accordance with another embodiment of the invention, and Fig. 17B illustrates the polarities of electrodes in a single phase of a pentapolar stimulus configuration in accordance with a further embodiment of the invention. In accordance with the principles described in detail in relation to Fig. 16, these multipolar configurations provide for current return to be shared between a larger number of return electrodes thus introducing a greater latitude to configure a biphasic or multiphasic stimulus in a manner which ensures that only one stimulus component gives rise to neural recruitment.

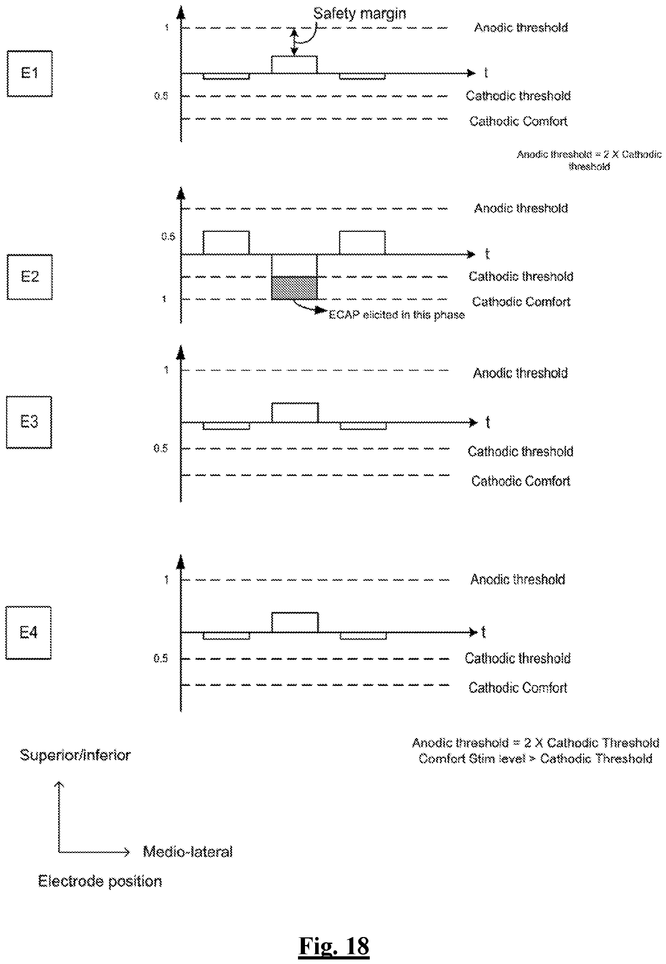

For example, Fig. 18 illustrates a triphasic quadrupolar stimulus configuration in accordance with yet another embodiment of the invention. This configuration permits the cathodic stimulus components delivered by electrodes E1, E3 and E4 in the first and third phases to have an amplitude which is only one sixth the amplitude of the supra-threshold stimulus component delivered by electrode E2 in the second phase. This provides additional operational freedom in selecting the amplitude of the single supra-threshold stimulus component, while maintaining all other stimulus components below threshold. The pentapolar configuration of Fig. 17B may be exploited to provide a one eighth amplitude in triphasic, while increasing the number of phases may additionally or alternatively be exploited to provide further operational freedom in selecting the amplitude of the single supra-threshold stimulus component.

It will be appreciated by persons skilled in the art that numerous variations and/or modifications may be made to the invention as shown in the specific embodiments without departing from the scope of the invention as defined in the appended claims.

## Claims

1. A neurostimulation device (100) comprising
at least three stimulation electrodes (150) configured to deliver an electrical stimulus to neural tissue; **characterised by**
a control unit (110) configured to deliver a first stimulus phase in which a first stimulus electrode delivers a supra-threshold stimulus component, returned by at least two other of the stimulation electrodes, the control unit (110) further configured to deliver at least a second stimulus phase in which at least two of the stimulus electrodes deliver a sub-threshold stimulus component, returned by the first stimulus electrode.

2. The device of claim 1 wherein the electrical stimulus comprises at least three phases, delivered by at least three electrodes, and configured so that only one stimulus component from one electrode in one of the phases is a supra-threshold cathodic stimulus component.

3. The device of claim 2 wherein the supra-threshold cathodic stimulus component is delivered in the final phase of the stimulus.

4. The device of any one of claims 1 to 3 configured to utilise four stimulus electrodes to deliver quadrupolar stimulation, and further preferably configured to deliver offset quadrupolar stimulation whereby the supra-threshold stimulus component is delivered by one electrode and returned by three electrodes.

5. The device of any one of claims 1 to 4 further configured to adaptively select a number of stimulus electrodes in order to best minimise second cathode stimulation by switching between multipolar stimulation modes.

6. The device of any one of claims 1 to 5 wherein a current borne by the first stimulus electrode is returned equally between the two or more other stimulus electrodes.

7. The device of any one of claims 1 to 5 wherein a current borne by the first stimulus electrode is returned unequally between the two or more other stimulus electrodes, preferably wherein unequal currents carried by the two or more other stimulus electrodes are configured to take an inequality which minimises stimulus artefact at a recording electrode.

8. The device of any one of claims 1 to 7, comprising a respective current source for each electrode configured to drive the desired current through that respective electrode in each phase of the stimulus.

9. The device of any one of claims 1 to 7, comprising a respective current source between adjacent pairs of stimulus electrodes to effect differential drive of the desired current through each electrode.

10. The device of any one of claims 1 to 9, configured to selectively connect one or more of the stimulus electrodes directly to a supply rail to serve as a passive return electrode.

11. The device of any one of claims 1 to 10, wherein the first stimulus electrode is interposed between the two or more other electrodes.

12. The device of any one of claims 1 to 10, wherein the first stimulus electrode is positioned to one side of both or all of the return electrodes.

13. The device of any one of claims 1 to 12, further comprising recording electrodes and measurement circuitry configured to obtain one or more recordings of a neural response evoked by the stimulus, and further configured to assess the one or more recordings of the neural response and to identify whether second cathode stimulation is occurring.

14. The device of claim 13 configured to apply stimuli of constant amplitude but having one or more distinct interphase gaps, observe the ECAPs evoked by each such stimulus, and compare the morphology of each such observed ECAP, to detect indicia of second cathode stimulation.

15. The device of claim 13 or claim 14 configured to detect second cathode stimulation by applying stimuli of constant amplitude but having distinct stimulus phase sequencing, observe the ECAPs evoked by each such stimulus, and compare the morphology of each such observed ECAP, to detect indicia of second cathode stimulation.

16. The device of any one of claims 13 to 15 further configured to use the outcome of second cathode stimulation to adjust a stimulus paradigm to seek prevention or reduction of second cathode stimulation when detected.

17. The device of any one of claims 13 to 16, further configured to carry out automated stimulus amplitude determination by:
applying stimuli at sub-threshold and supra-threshold amplitudes andobserving respective evoked ECAP responses,
determining therefrom a stimulus amplitude threshold at which ECAP responses are first evoked, and
setting the ongoing therapeutic stimulusamplitude as a multiple of the stimulus amplitude threshold,
preferably further configured for automated stimulus amplitude determination, by occasionally applying stimuli at sub-threshold and supra-threshold amplitudes and observing evoked ECAP responses, determining therefrom a stimulus amplitude threshold at which ECAP responses are first evoked, and setting the ongoing therapeutic stimulus amplitude as a multiple of the stimulus amplitude threshold.

18. The device of claim 17 wherein the ongoing therapeutic stimulus amplitude as set as a multiple within the range of 1.05 to 1.8 of the stimulus amplitude threshold, preferably configured to set the ongoing therapeutic stimulus amplitude as a multiple of 1.2 times the stimulus amplitude threshold.

## Patentansprüche

1. Neurostimulationsvorrichtung (100), umfassend:
wenigstens drei Stimulationselektroden (150), die konfiguriert sind, um einen elektrischen Reiz an Nervengewebe abzugeben, **gekennzeichnet durch**
eine Steuereinheit (110), die konfiguriert ist, um eine erste Reizphase zuzuführen, in der eine erste Stimulationselektrode eine überschwellige Reizkomponente zuführt, die von wenigstens zwei anderen der Stimulationselektroden zurückgeleitet wird, wobei die Steuereinheit (110) ferner konfiguriert ist, um wenigstens eine zweite Reizphase zuzuführen, in der wenigstens zwei der Stimulationselektroden eine unterschwellige Reizkomponente zuführen, die von der ersten Stimulationselektrode zurückgeleitet wird.

2. Vorrichtung nach Anspruch 1, wobei der elektrische Reiz wenigstens drei Phasen umfasst, die von wenigstens drei Elektroden zugeführt werden, und so konfiguriert, dass nur eine Reizkomponente von einer Elektrode in einer der Phasen eine überschwellige kathodische Reizkomponente ist.

3. Vorrichtung nach Anspruch 2, wobei die überschwellige kathodische Reizkomponente in der Endphase des Reizes zugeführt wird.

4. Vorrichtung gemäß einem der Ansprüche 1 bis 3, die konfiguriert ist, um vier Stimulationselektroden zum Zuführen einer quadrupolaren Stimulation zu verwenden, und die ferner vorzugsweise konfiguriert ist, um eine quadrupolare Offset-Stimulation zuzuführen, wobei die überschwellige Reizkomponente von einer Elektrode zugeführt und von drei Elektroden zurückgeleitet wird.

5. Vorrichtung nach einem der Ansprüche 1 bis 4, die ferner konfiguriert ist, um adaptiv eine Anzahl von Stimulationselektroden auszuwählen, um eine zweite Kathodenstimulation bestmöglich zu minimieren, indem zwischen multipolaren Stimulationsmodi umgeschaltet wird.

6. Vorrichtung nach einem der Ansprüche 1 bis 5, wobei ein von der ersten Stimulationselektrode geführter Strom zu gleichen Teilen von den zwei oder mehr anderen Stimulationselektroden zurückgeleitet wird.

7. Vorrichtung nach einem der Ansprüche 1 bis 5, wobei ein von der ersten Stimulationselektrode geführter Strom zu unterschiedlichen Teilen von den zwei oder mehr anderen Stimulationselektroden zurückgeleitet wird, vorzugsweise wobei ungleiche Ströme, die von den zwei oder mehr anderen Stimulationselektroden geführt werden, so konfiguriert sind, dass sie eine Ungleichheit annehmen, die die Reizartefakte an einer Aufzeichnungselektrode minimiert.

8. Vorrichtung nach einem der Ansprüche 1 bis 7, umfassend für jede Elektrode eine eigene Stromquelle, die konfiguriert ist, um den erwünschten Strom durch diese jeweilige Elektrode in jeder Phase des Reizes zu leiten.

9. Vorrichtung nach einem der Ansprüche 1 bis 7, umfassend jeweils eine Stromquelle zwischen benachbarten Stimulationselektrodenpaaren, um eine differentielle Leitung des erwünschten Stroms durch jede Elektrode zu bewirken.

10. Vorrichtung nach einem der Ansprüche 1 bis 9, die so konfiguriert ist, dass eine oder mehrere der Stimulationselektroden selektiv direkt an eine Versorgungsschiene angeschlossen werden können, um als passive Gegenelektrode zu dienen.

11. Vorrichtung nach einem der Ansprüche 1 bis 10, wobei die erste Stimulationselektrode zwischen den zwei oder mehr anderen Elektroden angeordnet ist.

12. Vorrichtung nach einem der Ansprüche 1 bis 10, wobei die erste Stimulationselektrode an einer Seite von beiden oder allen Gegenelektroden angebracht ist.

13. Vorrichtung nach einem der Ansprüche 1 bis 12, umfassend ferner Aufzeichnungselektroden und Messschaltungen, die so konfiguriert sind, dass eine oder mehrere Aufzeichnungen einer durch den Reiz evozierten neuralen Reaktion erhalten werden können, und die ferner konfiguriert sind, um eine oder mehrere Aufzeichnungen der neuralen Reaktion auszuwerten und festzustellen, ob eine zweite Kathodenstimulation stattfindet.

14. Vorrichtung nach Anspruch 13, die konfiguriert ist, um Reize mit konstanter Amplitude anzulegen, die jedoch eine oder mehrere eindeutige Interphasenlücken aufweisen, die durch einen jeden solchen Reiz evozierten ECAPs zu beobachten, und die Morphologie eines jeden solchen beobachteten ECAP zu vergleichen, um Indizien für eine zweite Kathodenstimulation zu erkennen.

15. Vorrichtung nach Anspruch 13 oder Anspruch 14, die konfiguriert ist, um eine zweite Kathodenstimulation zu erkennen, indem Reize mit konstanter Amplitude angelegt werden, die jedoch eine eindeutige Reizphasensequenzierung besitzen, die durch einen jeden solchen Reiz evozierten ECAPs beobachtet werden und die Morphologie eines jeden solchen beobachteten ECAP verglichen wird, um Anzeichen eine zweite Kathodenstimulation zu erkennen.

16. Vorrichtung nach einem der Ansprüche 13 bis 15, die ferner konfiguriert ist, um das Ergebnis der zweiten Kathodenstimulation zu verwenden, um ein Stimulusparadigma anzupassen, um die Verhinderung oder Verringerung einer zweiten Kathodenstimulation anzustreben, wenn diese erkannt wird.

17. Vorrichtung nach einem der Ansprüche 13 bis 16, die ferner konfiguriert ist, um eine automatisierte Reizamplitudenbestimmung durchzuführen durch:
Anlegen von Reizen mit unterschwelligen und überschwelligen Amplituden und Beobachten der jeweils evozierten ECAP-Reaktionen,
Bestimmen daraus einer Reizamplitudenschwelle, bei der ECAP-Reaktionen erstmalig evoziert werden, und
Einstellen der Amplitude des laufenden therapeutischen Reizes als Vielfaches der Reizamplitudenschwelle,
und die vorzugsweise ferner konfiguriert ist für eine automatisierte Reizamplitudenbestimmung durch gelegentliches Anlegen von Reizen mit unterschwelligen und überschwelligen Amplituden und Beobachten der evozierten ECAP-Reaktionen, Bestimmen daraus einer Reizamplitudenschwelle, bei der ECAP-Reaktionen erstmalig evoziert werden, und Einstellen der Amplitude des laufenden therapeutischen Reizes auf ein Vielfaches der Reizamplitudenschwelle.

18. Vorrichtung nach Anspruch 17, wobei die Amplitude des laufenden therapeutischen Reizes auf das 1,05- bis 1,8-fache Vielfache der Reizamplitudenschwelle eingestellt wird, vorzugsweise konfiguriert, um die Amplitude des laufenden therapeutischen Reizes auf das 1,2-fache Vielfache der Reizamplitudenschwelle einzustellen.

## Revendications

1. Dispositif de neurostimulation (100) comprenant :
au moins trois électrodes de stimulation (150) configurées pour délivrer un stimulus électrique à un tissu nerveux ; **caractérisé par**
une unité de commande (110) configurée pour délivrer une première phase de stimulus dans laquelle une première électrode de stimulus délivre une composante de stimulus supra-seuil, renvoyée par au moins deux autres des électrodes de stimulation, l'unité de commande (110) étant en outre configurée pour délivrer au moins une seconde phase de stimulus dans laquelle au moins deux des électrodes de stimulus délivrent une composante de stimulus sous-seuil, renvoyée par la première électrode de stimulus.

2. Dispositif selon la revendication 1, dans lequel le stimulus électrique comprend au moins trois phases, délivrées par au moins trois électrodes, et configuré de telle sorte qu'une seule composante de stimulus provenant d'une électrode dans l'une des phases est une composante de stimulus cathodique supra-seuil.

3. Dispositif selon la revendication 2, dans lequel la composante de stimulus cathodique supra-seuil est délivrée dans la phase finale du stimulus.

4. Dispositif selon l'une quelconque des revendications 1 à 3, configuré pour utiliser quatre électrodes de stimulus afin de délivrer une stimulation quadripolaire, et en outre de préférence configuré pour délivrer une stimulation quadripolaire décalée moyennant quoi la composante de stimulus supra-seuil est délivrée par une électrode et renvoyée par trois électrodes.

5. Dispositif selon l'une quelconque des revendications 1 à 4, configuré en outre pour sélectionner de manière adaptative un nombre d'électrodes de stimulus afin de minimiser au mieux une seconde stimulation de cathode en passant d'un mode de stimulation multipolaire à l'autre.

6. Dispositif selon l'une quelconque des revendications 1 à 5, dans lequel un courant porté par la première électrode de stimulus est renvoyé de manière égale entre les deux autres électrodes de stimulus ou plus.

7. Dispositif selon l'une quelconque des revendications 1 à 5, dans lequel un courant porté par la première électrode de stimulus est renvoyé de manière inégale entre les deux autres électrodes de stimulus ou plus, de préférence dans lequel des courants inégaux véhiculés par les deux autres électrodes de stimulus ou plus sont configurés pour prendre une inégalité qui minimise l'artefact de stimulus au niveau d'une électrode d'enregistrement.

8. Dispositif selon l'une quelconque des revendications 1 à 7, comprenant une source de courant respective pour chaque électrode configurée pour piloter le courant souhaité à travers cette électrode respective dans chaque phase du stimulus.

9. Dispositif selon l'une quelconque des revendications 1 à 7, comprenant une source de courant respective entre des paires adjacentes d'électrodes de stimulus afin d'effectuer un pilotage différentiel du courant souhaité à travers chaque électrode.

10. Dispositif selon l'une quelconque des revendications 1 à 9, configuré pour connecter sélectivement une ou plusieurs des électrodes de stimulus directement à un rail d'alimentation pour servir d'électrode de retour passive.

11. Dispositif selon l'une quelconque des revendications 1 à 10, dans lequel la première électrode de stimulus est interposée entre les deux autres électrodes ou plus.

12. Dispositif selon l'une quelconque des revendications 1 à 10, dans lequel la première électrode de stimulus est positionnée sur un côté de deux ou de l'ensemble des électrodes de retour.

13. Dispositif selon l'une quelconque des revendications 1 à 12, comprenant en outre des électrodes d'enregistrement et une circuiterie de mesure configurée pour obtenir un ou plusieurs enregistrements d'une réponse neurale évoquée par le stimulus, et configurée en outre pour évaluer les un ou plusieurs enregistrements de la réponse neurale et pour identifier si une seconde stimulation de cathode se produit.

14. Dispositif selon la revendication 13, configuré pour appliquer des stimuli d'amplitude constante mais ayant un ou plusieurs espaces interphasiques distincts, observer les ECAPs évoqués par chacun de ces stimuli, et comparer la morphologie de chacun de ces ECAPs observés, pour détecter des indices d'une seconde stimulation de cathode.

15. Dispositif selon la revendication 13 ou la revendication 14, configuré pour détecter une seconde stimulation de cathode en appliquant des stimuli d'amplitude constante mais ayant des séquences de phase de stimulus distinctes, observer les ECAPs évoqués par chacun de ces stimuli, et comparer la morphologie de chacune de ces ECAPs observés, pour détecter des indices d'une seconde stimulation de cathode.

16. Dispositif selon l'une quelconque des revendications 13 à 15, configuré en outre pour utiliser le résultat d'une seconde stimulation de cathode pour ajuster un paradigme de stimulus afin de rechercher la prévention ou la réduction d'une seconde stimulation de cathode lorsqu'elle est détectée.

17. Dispositif selon l'une quelconque des revendications 13 à 16, configuré en outre pour réaliser une détermination automatisée d'amplitude de stimulus par :
l'application de stimuli à des amplitudes sous-seuil et supra-seuil et l'observation des réponses ECAP évoquées respectives,
la détermination, à partir de cela, d'un seuil d'amplitude de stimulus auquel des réponses ECAP sont évoquées pour la première fois, et
le réglage de l'amplitude de stimulus thérapeutique en cours comme un multiple du seuil d'amplitude de stimulus,
de préférence configuré en outre pour la détermination automatisée d'amplitude de stimulus, par l'application occasionnelle de stimuli à des amplitudes sous-seuil et supra-seuil et l'observation des réponses ECAP évoquées, par la détermination, à partir de cela, d'un seuil d'amplitude de stimulus auquel des réponses ECAP sont évoquées pour la première fois, et par le réglage de l'amplitude de stimulus thérapeutique en cours comme un multiple du seuil d'amplitude de stimulus.

18. Dispositif selon la revendication 17, dans lequel l'amplitude de stimulus thérapeutique en cours étant réglée comme un multiple dans la plage de 1,05 à 1,8 du seuil d'amplitude de stimulus, de préférence configuré pour régler l'amplitude de stimulus thérapeutique en cours comme un multiple de 1,2 fois le seuil d'amplitude de stimulus.
